# EUROPEAN PATENT APPLICATION

(11) **EP 4 651 148 A1**
(43) Date of publication of application: **19.11.2025**
(21) Application number: 25176062.5
(22) Date of filing: 13.05.2025
(51) Int. Cl.: G16H 30/40, G16H 50/20

(54) **GENERATIVE ARTIFICIAL INTELLIGENCE FOR DECISION MAKING IN MEDICAL IMAGING**

(30) Priority: 16.05.2024 US 202418665857
(71) Applicant: Siemens Medical Solutions USA, Inc., Malvern, PA 19355 (US)
(72) Inventor: ZHANG, Yue, Short Hills, NJ 07078 (US); SHARMA, Puneet, Princeton Junction, NJ 08550 (US); GAO, Riqiang, Plainsboro, NJ 08536 (US); PASSERINI, Tiziano, Plainsboro, NJ 08536 (US)
(74) Representative: HKW Intellectual Property PartG mbB

(57) **Abstract**

For decision making in medical image processing, a large language model (LLM) artificial intelligence (AI) (602) generates a program (604) calling a series of available features to answer a user request. Rather than navigating through various functions (606) in the GUI, the user may input a question, and the LLM AI (602) then programs the medical imaging system to implement the functions (606) to answer the question.

## Description

### BACKGROUND

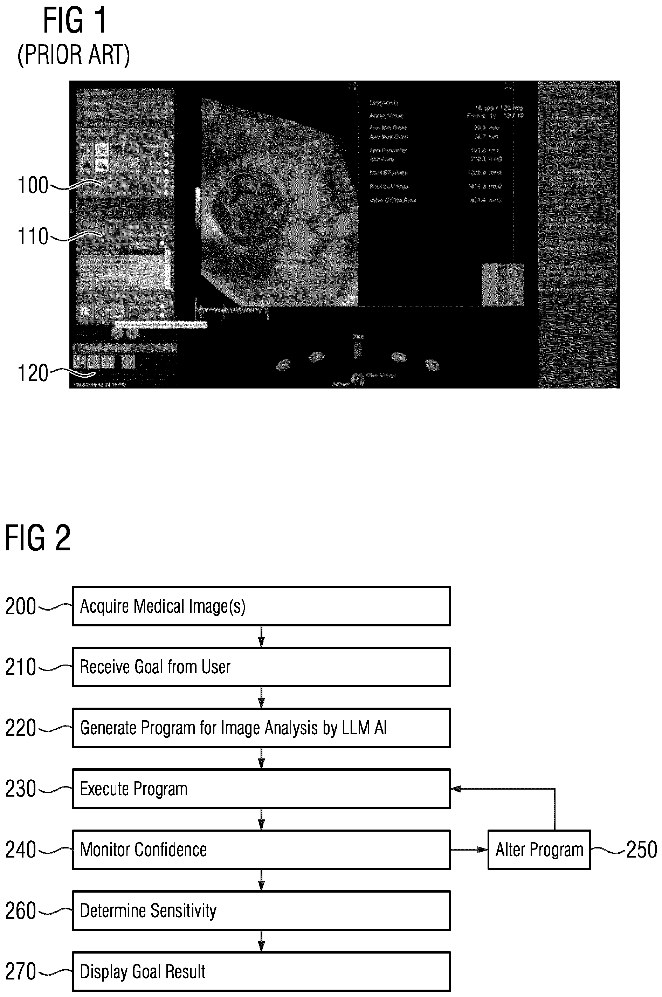

The present embodiments relate to decision making in medical imaging. For example, medical imaging is used for structural heart interventions. Structural heart interventions may mitigate the burdens associated with traditional open-heart surgeries, particularly for patients who are considered high-risk candidates. Various medical imaging features and software have been developed for structural heart intervention. For example, Siemens Heathineers developed software including eSie Valves^{©} and syngo TrueFusion^{©}, which offer numerous imaging and automation features, including automatic structure detetection, segmentation, quantification, and visualization. Figure 1 shows an example graphics user interface image with various selections for features for eSie Valves^{©} 100, annulus measurement 110, and movie controls 120, where other general functions (panels) and corresponding features are available for selection. The user decides which features to use for a given intervention.

In the graphical user interface (GUI), these features are represented by a rich number of digital buttons, spreaded over a number of virtual panels. With more and more advanced features being developed, the GUI will potentially grow crowded, reducing the usability, increasing search time by the user, and eventually creating a negative impact on the operation efficiency. Time may be crucial in cardiac intervention, but the large selection of features may cause delay. The large number of available features may unavoidably bring more and more operation stress to the user, potentially causing distraction and interruption, creating a negative impact on the operation efficiency.

There are existing approaches and concepts that try to make software easier to use. For example, a verbal command or a search window may quickly locate a feature (e.g., panel, button or function). However, a single feature is found and used at a given time.

### SUMMARY

Systems, methods, and instructions on computer readable media are provided for decision making in medical image processing. A large language model (LLM) artificial intelligence (AI) generates a program calling a series of available features to answer a user request. Rather than navigating through various functions in the GUI, the user may input a question, and the LLM AI then programs the medical imaging system to implement the functions to answer the question.

In a first aspect, a method is provided for decision making in a medical imaging system. A first medical image of a patient is acquired. A LLM AI receives user input identifying a goal with respect to the first medical image. The LLM AI generates an executable program calling multiple analysis functions of the medical imaging system to achieve the goal. An image processor of the medical imaging system executes the executable program. At least a first one of the multiple analysis functions called by the executing of the executable program operates on the first medical image. An estimate of the goal is displayed. The estimate is derived from results of the executing.

In a second aspect, a medical system is provided. A memory is configured to store a large language model artificial intelligence (LLM AI) calibrated for medical imaging. A user input is configured to receive a sentence defining a user request with respect to a medical image of a patient. A processor is configured to input the sentence to the LLM AI, to receive a sequence of calls for application programming interfaces from the LLM AI generated in response to the input, and to implement the sequence using the medical image. A display is configured to display an answer to the user request derived from the implementation of the sequence.

In a third aspect, a method is provided for decision making in a medical imaging system. The medical imaging system is programmed by a large language model to operate on medical images of different modalities using available functions of the medical imaging system to answer a user request. An answer to the user request is displayed.

Any one or more of the aspects or concepts summarized above or in the Illustrative Embodiments below may be used alone or in combination. The aspects or concepts described for one Illustrative Embodiment or aspect may be used in other embodiments or aspects. The aspects or concepts described for a method or system may be used in others of a system, method, or non-transitory computer readable storage medium.

These and other aspects, features, embodiments, and advantages will become apparent from the following detailed description of preferred embodiments, which is to be read in connection with the accompanying drawings. The present invention is defined by the following claims, and nothing in this section should be taken as a limitation on those claims. Further aspects and advantages of the invention are discussed below in conjunction with the preferred embodiments and may be later claimed independently or in combination.

### BRIEF DESCRIPTION OF THE DRAWINGS

The components and the figures are not necessarily to scale, emphasis instead being placed upon illustrating the principles of the embodiments. Moreover, in the figures, like reference numerals designate corresponding parts throughout the different views.
Figure 1 is a GUI for a medical imaging system;
Figure 2 is a flow chart diagram of one embodiment of a method for decision making using LLM AI in a medical imaging system;
Figure 3 illustrates example functions, existing workflow to manually select and use a sequence of functions, and a proposed workflow to select and use a sequence of functions using LLM AI;
Figure 4 is a flow chart diagram of another embodiment of the method for decision making in a medical imaging system;
Figure 5 illustrates an example program code generated by LLM AI;
Figure 6 is a block diagram of one embodiment of a medical imaging system using LLM AI for programming for a patient; and
Figure 7 is a block diagram of an example AI.

### DETAILED DESCRIPTION OF EMBODIMENTS

A generative AI-driven approach is provided for enhanced decision-making in single- and multi-modal medical imaging, such as in image processing for structural heart interventions. A LLM-based generative AI automatically generates an excutable program and/or code based on user text input and acquired single or multi modal image(s). Compared with existing approaches which are search based, the proposed approach is generative. Both existing features and/or application programming interfaces (APIs) may be called as well as generating a new hybrid function with a stack of existing function APIs for a target task. The hybrid function did not previously exist in the system but is instead generated at needed based on the user input. LLM is leveraged to automatically program in single- and/or multi-modal clinical medical applications.

In a further approach, each step of excution is self-diangosed for the safety of use of the clinical software. The LLM AI or processor monitors the confidence of the APIs. When the confidence of an API is low, the LLM AI iterates with the user for risk management. Instead of a sinle command pass, the interaction between the user and LLM AI is used to alter the hybrid function whenever low confidence results or ambiguity is presented. This interaction adapts the hybrid function to more likely provide the information desired by the user.

Figure 2 shows an example implementation of a method for decision making in a medical imaging system. A generative AI-driven system takes single-or multi-modal image(s) and a pool of all available APIs as input to automatically generate an executable program to achieve user's request for a patient. The method includes a risk management feature. During program execution, whenever an intermediate result is generated with low confidence, the generative AI iterates with the user for clarification and instruction.

The method is performed in the order shown (e.g., top to bottom or numerical), but other orders may be used. For example, act 210 may be performed before act 200. As another example, act 260 may be performed prior to act 240.

Additional, different, or fewer acts may be provided. For example, acts 240, 250, and/or 260 are not performed. In another example, act 270 is not performed, such as where the results are stored or transferred. As another example, acts for scanning the patient and/or using the goal result are provided.

The method is performed by a medical imaging system, such as a medical scanner, a workstation, a server, a computer, or a processor for operating on medical images. A memory stores the LLM AI, and a processor applies the LLM AI. A user input is provided for user interaction with the LLM AI. The same or different processor implements functions called by a program generated by the LLM AI. The processor(s) perform various acts to acquire, receive, generate, execute, monitor, alter, and/or determine. A display is used to display results. Other devices, circuits, or equipment may be used.

In act 200, the processor acquires one or more medical images of a patient. The medical image or images are acquired by loading from memory (e.g., from a picture archiving and communications system or patient medical record), transfer over a computer network, and/or by scanning the patient.

The medical images are ultrasound, magnetic resonance (MR), computed tomography (CT), single photon emission computed tomography (SPECT), positron emission tomography (PET), fluoroscopy, angiography, other x-ray, and/or another type of medical image. In one approach, one or more medical images of the patient using one modality (e.g., x-ray) are acquired. In another approach, images from different modalities are acquired, such as CT and ultrasound. The images from the different medical imaging modalities may be from scanning the patient at a same time or different times. One modality (e.g., CT, MR, PET, and/or SPECT) may be pre-operative images represented a three-dimensional volume of the patient, and another modality (e.g., x-ray or ultrasound) may be images acquired by scanning during an interventional procedure. Different modalities may be images from scanning during an intervention.

In one approach, the images are for a medical intervention procedure, such as structural heart intervention. Images for other interventions and/or for diagnosis may be received. The structural heart intervention example is used herein.

In act 210, the processor receives user input. The user input identifies a goal with respect to the medical images. The input is received from a graphics user interface, such as from a microphone, keyboard, trackball, touch screen, touch pad, and/or mouse. The user input is alphanumeric text and/or audio. Audio input may be converted to alphanumeric text. The user can interact with the proposed system in any of various ways. For example, the user could use voice to issue a request or could choose from a list of options that are shown to the user on a screen. As another examle, the user may type text.

The received input is a question, order, or other statement in a sentence structure. The input is a full sentence or a clause. The input may be multiple sentences. Any sentence structure may be used, including bullet points. In one approach, the input is conversational, such as an indication of the goal written out. The goal may be a desired measurement to be made, comparison, identification, or other information useful for medical intervention or diagnosis. Any input appropriate for a LLM AI is received. The input indicates the information desired by the user.

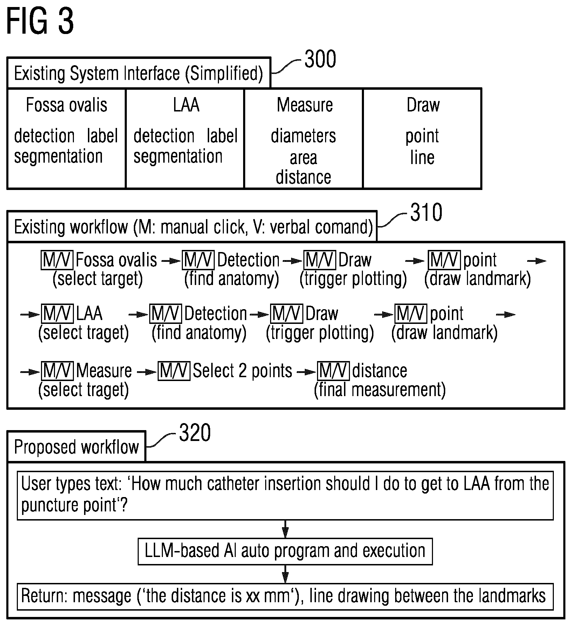

Figure 3 shows an example input in sentence structure as compared to input by separate, manual activation of a sequence of functions or application programming interfaces (APIs). The medical imaging system has a variety of functions to assist in analyzing an image or images for a structural heart intervention. The medical imaging system interface (e.g., GUI) 300 includes buttons, menus, or other selection options for various functions, such as (1) detection, labeling, and/or segmentation of fossa ovalis, (2) detection, labeling, and/or segmentation of left arterial appendage (LAA), (3) measurement of diameter, area, or distance, and (4) drawing of a point or line. Other functions may be provided for the listed options, and/or other options of types of functions and/or functions related to specific anatomy may be provided. The medical imaging system interface 300 provides for access to all the functions (e.g., APIs) available to a given medical imaging system.

In the example of Figure 3, the physician is performing LAA occlusion. The physician needs to determine the traveling distance between transeptal punctual point to LAA ostium. In the existing workflow 310, this is done by manually or verbally searching for and calling each of a long sequence of commands (i.e., functions or APIs) shown by the arrows. The functions or APIs are called one-by-one by the physician. This can be time consuming. Even though all these calls could be programmed into another new button on the GUI 300, due to the complexities of the procedure and other procedures, there will be numerous 'new buttons' created if one targets to fit all the potential needs from the physician. For example, to be aligned with this existing case, the physician may instead measure FO center to LAA neck, LAA ostium to LAA neck, etc. The system can then become cumbersome.

In comparison, the proposed workflow 320 using the LMM AI recieves a request in sentence structure. For examle, the user types "How much catheter insertion should I do to get to LAA from the puncture point?" The same entry may be phrased differently, such as by a different physian, as "Tell me the distance from the puncture point to the LAA." Any of various goal statements in sentence structure may be received. The LMM AI understands the LAA procedure context, so will 1) interprete the ,puncture point' as ,a point on fossa ovalis,' 2) automatically call the corresponding sequence of commands in the right order to finish the task, and 3) return a message (e.g., "the distnace is xx mm") while adding a line drawing between the puncture point and the LAA on the image. This input provides an intuitive way for interaction of the physician with the medical imaigng system. The proposed workflow 320, using the LMM AI, understands the needs expressed in the input. For example, the word 'puncture point' is ambiguous, so the LMM AI understands the semantic environment to identify the fossa ovalis as the puncture point and automatically program on-the-fly to finish the task.

The processor receives the input. The processor implements the LLM AI, so the LLM AI receives the user input identifying the goal with respect to the medical image(s) of the patient.

The LLM AI is any now known or later developed LLM, such as GPT (e.g., CHATGPT) by OPENAI, PALM or GEMINI by GOOGLE, XAI by GROK, LLAMA by META, CLAUDE by ANTHROPIC, DBRX by DATABRICK, or another LLM. In one approach, the LLM AI is a transformer formed by a set of neural networks that consist of an encoder and a decoder with self-attention capabilities. In another approach, the LLM AI is an architecture with transformer decoder-only. As another approach, the LLM AI uses a recurrent neural network and/or a state space model. The LLM AI acquires knowledge about syntax, semantics, and ontology in human language corpora through machine learning. LLMs harness vast language datasets to generate human-like text and engage in natural language understanding. When integrated with chain-of-thought prompting, these models gain the ability to connect disparate pieces of information coherently, forming a structured, logical narrative. This approach fosters more contextually aware and insightful responses, bridging the gap between traditional rule-based AI and the nuanced understanding of human language. Additionally, the convergence of LLMs and chain-of-thought prompting aligns with the principles of neurosymbolic programming, where symbolic reasoning and neural networks harmonize, enabling AI systems to grasp abstract concepts and perform complex tasks with a deeper understanding of the underlying semantics.

The LLM AI is used to automatically program. Various LLM AI systems may be converted for use in medical imaging to program. TOOLFORMER is a model trained to call a single API based on text inputs. VISPROG uses in-context learning ability of GPT3 to execute a sequence of programs with a single modal image (RGB) input. TOOLLLM prompts CHATGPT to generate human instructions to use APIs from an open API hub RAPIDAPI, then ask CHATGPT to search for valid sequence of API calls for each instruction. The LLM AI is used in the medical context to automatically call a sequence of APIs for complex medical imaging tasks. The LLM AI uses neurosymbolic programming to generate a program in response to receipt of the user input.

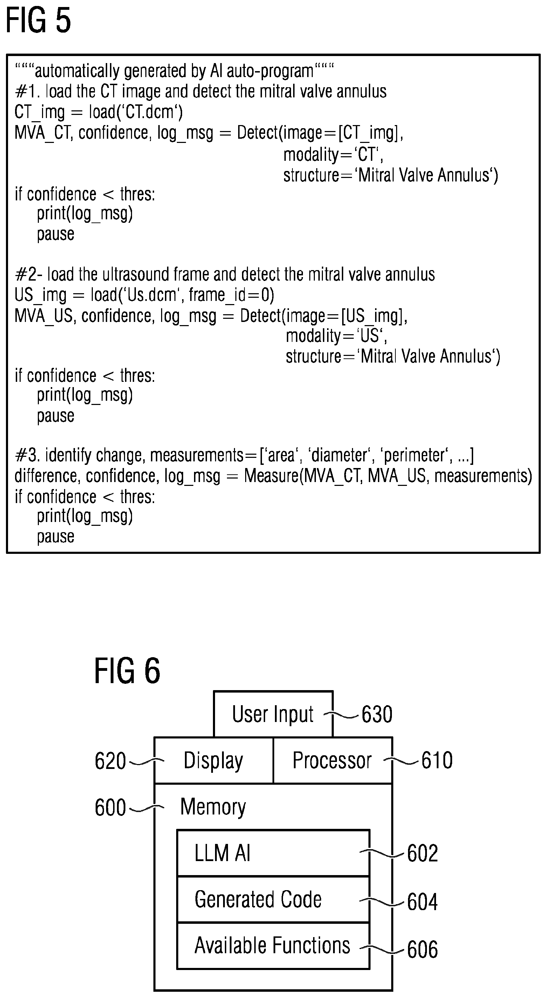

The generated program is executable, such as being computer code or code implements by the processor. Figure 5 shows an example where the LLM AI generates the code to load a CT image and an ultrasound image and to make measurements in the two images. The generated computer code or executable program does not otherwise exist in the medical imaging system. There is no single executable that can be called in the medical imaging system prior to generation by the LLM AI.

The LLM AI generates an executable program calling multiple analysis functions of the medical images system, such as an executable program calling the functions listed in the existing workflow 310 in the order shown in response to the input of the proposed workflow 320 without the manual one-by-one selection of the existing workflow 310. The LLM AI selects the list of functions (e.g., API) and order of those functions in an output as executable code to achieve the input goal. One or more of the available functions of the medical imaging system are used by the LLM AI programming to answer the user request. Fewer than all available functions are called by the generated executable program. The LLM AI selects the functions to be included in the executable code from the available functions, such as selecting a sub-set. Some of the function (e.g., API) are for image processing, such as for loading an image, detecting a landmark in an image, segmenting anatomy, tracking movement, measuring with respect to one or more landmarks, and/or drawing on the medical image. Different or the same functions may be provided for images of different modalities. For example, a function for the same purpose (e.g., detecting a specific landmark) for one modality is implemented by a different function for another modality. The LLM AI generates the executable program with calls for functions for the appropriate types (modalities) of input images. The functions are ordered in a logical manner so that each function receives as input the information needed to operate. The LLM AI generates the executable program to include the needed functions in the proper order.

The LLM AI operates in the medical imaging context. Rather than using a LLM AI without context training, the LLM AI is engineered and/or calibrated for operation in the medical context. The context may be procedure specific or generalized over multiple different procedures, such as structural heart interventions.

In one approach, the LLM AI is prompt-engineered. A database of workflow examples of uses of the medical imaging system and available analysis functions of the medical imaging system are used. The LLM AI is prompted to review the database to acquire knowledge about semantics, syntax, and terms (e.g., ontology) for the medical imaging context. The LLM AI may be prompted with ground truth examples from a prompt describing the database, an instruction to generate the executable program in response to inputs, and any limitations to be followed.

For example, the LLM AI is calibrated as a dedicated module for workflow in the medical imaging context using in-context learning. A dataset of workflow knowledge is used, such as database including strutural heart intervention textbooks, protocols, papers, and/or transcripts with operation records from examples of the procedure being performed (e.g., expert curration from video recordings). The LLM AI is asked to read through the dataset, and populate all questions and commands (up to N, e.g. 10K) related to medical image processing. Next, a dataset of APIs (e.g., documents including all available function names as well as their use cases), together with examples of use of the APIs is acquired. The LLM AI is asked to read through this API dataset to gain knowledge of the available APIs.

An instruction is generated for the prompt engineering. Groud truth examples are then generated using a prompt. A prompt template is provided to the LLM AI to instruct the LLM AI what to do with the database information. For example, the prompt is:
You will be provided with a list of API functions, the descriptions of these API functions, and the parameters required for each API function and examples of how to use them. Your task is to generate a sequence of API calls according to the protocols in the previous given knowledge dataset. For any API function that will return a confidence score, you need to check whether that score is below a given threshold. If so, you need to print the log message from that function, pause the executing, and wait for the user's instruction to either to continue or stop. You can only use APIs from the dataset of APIs. If any parameter of the API is not specified, you should use the default parameter instead of an arbitrary one. Return the final output and log message to the user when the sequence calls of the APIs execute successfully.
Other prompts with more or less instructions may be used. The LLM AI is given instructions for what is to be done with what and how. The prompt calibrates the LLM AI to the medical imaging context.

Other types of learning context for LLM AI may be used. For example, reinforcement learning based on human feedback (RLHF) (e.g., proximal policy optimization) is used. As another example, instructing tuning is used based on bootstrapping from human-generated corrections. In yet another example, a mixture of experts (MoE) process is used.

For calibration, the LLM AI is calibrated from (1) questions for workflow examples and (2) positive and/or negative feedback for example executable programs generated by the LLM AI for the questions. After prompting for the LLM AI to learn the context, the LLM AI is calibrated. Given the prompt, the LLM is asked to generate a sequence of API calls for each question pre-generated from the knowledge dataset and/or other questions. A database of images is collected, and executable programs generated by the LLM AI are executed on the images. The executable programs that are successful (e.g., successfully executed with desired results) are used as positive example feed back to the LLM AI. Negative examples (e.g., failure to execute and/or delivery of different results than desired) may be feed back as well or instead of positive examples. The LLM AI is further calibrated by learning from the positive and/or negative examples. Other calibration may be used.

Once the context is learned and/or the LLM AI is calibrated, the LLM AI is used for a specific patient. The image(s) of the patient are acquired in act 200, and the goal for those image(s) and patient is received in act 210. The LLM AI generates the executable program in act 220 based on the image(s) and/or goal.

In act 230 of Figure 2, the processor of the medical imaging system executes the executable program generated by the LLM AI. The coding or program is executed, resulting in calling the selected analysis funcitons in the selected order. One, some, or all the analysis functions available to the medical imaging system are called based on the LLM AI-created executable program.

One or more of the called functions (e.g., APIs) operates on one or more of the medical images of the patient. The function performs an action relative to or using the medical image. For example, the function loads the medical image, detects a landmark in the medical image, segments anatomy in the medical image, tracks anatomy, measures, drawings on, saves, and/or performs another action on the medical image. In the example of Figure 3, the executable program calls the fossa ovalis functions for detection, trigger plotting, draw on the image, calls the LAA functions for detection, trigger, and draw on the image, and calls the measurement functions for selecting two points and measuring distance on the image. The LLM AI generated program is executed to cause the various functions to be performed relative to one or more of the medical images. One or more functions may not operate on one, more, or all the medical images, such as a function to collect clinical data. Different or the same functions may be called to operate on different images.

In act 240, the processor monitors confidence information. One or more of the called analysis functions may generate a confidence. For example, analysis funcitons using a machine-learned model (e.g., AI) may output results including confidence. The processor monitors these confidence outputs during the execution of act 230. When one or more of the existing APIs has an output the quantifies the uncertainity or a confidence score for that specific task (e.g., AI output), the prossor implements a risk management feature based on the confidence.

The processor may be programmed to monitor. Alternatively, the LLM AI is prompted or instructed to monitor any confidence outputs. The LLM AI, implemented by the processor, monitors.

Each function may have a confidence threshold. Alternatively, a default confidence threshold is used for each function. The different functions may be use the same or different thresholds. The monitroing compares the confidence to the threshold for a given function. A cumulative confidence may be generated, such as by averaging confidences from multiple functions. The cummulative confidence may be compared to a threshold.

When a confidence is below the threshold (i.e., uncertainity above a threshold), the processor generates a warning. For examle, the LLM AI raises a warning to the user (e.g., text and/or audio) whenever low confidence intermediate (function) results are generated.

In act 250, the processor alters the executable program based on the confidence information. Where the confidence is below a threshold, the LLM AI stops the execution communicates with the user. The communication provides an interaction where the user can instruct or influence actions to take in response. For example, the user can request a different landmark to be found and used. The LLM AI and user iterate until all commands or functions are executed with high confidence. The alteration is performed in response to clarification and/or instructions from the user, which may be as simple as continuing. The LLM AI receives and interprets the communications from the user to alter the executable program.

The functions being used may be altered. Values of one or more parameters of a function may be altered. The order of the functions may be altered. Any alteration to the executable program may be made. The LLM AI alters, based at least in part, on the interaction with the user. The LLM AI rewrites or changes the executable program.

By monitoring in act 240, a risk management feature is provided. The executable program may be stopped or paused to manage risk. By altering in act 250, the risk management feature further provides for ways to provide high confidence in obtaining the goal. During program execution, whenever an intermediate (function) result is generated with low confidence, the LLM AI starts iterating with the user for clarification and instruction to alter the program.

Once the execution of act 230 is complete, an estimate of the goal input by the user in act 210 is generated. The executable program causes functions to calculate the desired information, such as the distance from the puncture point to the LAA. The estimate of that distance is output. The answer to the user request is determined or estimated.

In act 260, the processor determines a sensitivity of the estimate. By altering some aspect of the executable program, one or more functions, user input, and/or the input (e.g., image or images), the sensitivity of the estimate to the alteration is calculated. The risk management feature of the processor caclulates a confidence score by perturbing and analyzing the sensitivitiy of the underlying LLM output with respect to slight changes, such as in the user input.

Additionally, or alterantively, the processor determines an overall or aggregate confidence. The confidences output by the functions are combined to indicate a confidence in the estimate. Where a function may have varying confidence but one is not output, a default or study-based confidence may be used. Alternatively, that function does not contribute to the aggregate confidence for the estimate.

In act 270, the processor generates an image, and a display displays the image to the user. The image includes an estimate of the goal, such as text communicating the estimate in sentence structure. The image may be output to a display, into a patient medical record, and/or to a report.

The estimate is derived from the results of executing the program generated by the LLM AI. The estimate may be the result, such as a measure, detected landmark, or segmentated anatomy. The estimate may be calucated from the result of executing the program, such as a heart rate calcuated from results of tracking, a flow pattern modeled from anatomy over time detected as the results, or a diagnosis classified based on results.

An answer to the user request is displayed. The answer may be a graphic on the image, annotation on the image, text, graph, link to information, number, and/or another output. In alternative approaches, an audio output is generated, such as in conversation by the LLM AI.

Other information may be output as well. For example, the aggregate confidence is output with the estimate. As another example, the sensitivity is output with the estimate. In another example, results from component functions called by the executable program are output, such as the various measurements, detections, segmentations, tracking and/or other operations used to reach the goal. Values of parameters for the functions and/or confidence by function may also be output. The images used by the executable program may be displayed.

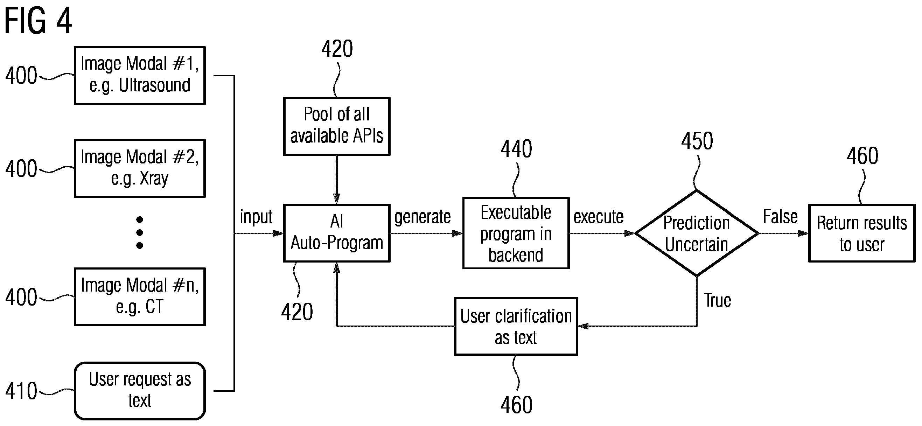

Figure 4 is a flow chart diagram of another implementation of the method for a LLM-based generative AI system. The LLM AI is implemented in a medical imaging system, such as a server, workstation, or computer for operating on or with medical images. The medical imaging system may be used for planning an intervention, during an intervention, and/or diagnosis. The medical imaging system may be or include a medical scanner or may receive images (e.g., scan data or display formated images) from one or more medical scanners (e.g., different modalities).

The LLM-based generative AI automatically generates 430 excutable program and/or code 440 based on user text input 410 and acquired single or multi modal image inputs 400. Given single or multi modal image acquisition and the user request 410 as input, the AI system checks all available function APIs 420 in the medical imaging system, and automatically generates 430 executable program (code) 440 in the backend. The system then executes 440 each line of the generated code.

Further, each step of excution is self-diagnosed by the LLM AI or processor with or without using the LLM AI based on predictions of uncertainty 450. Where the results of a called function are uncertain (e.g., unclear commands or low conficence resultes), the LLM AI iterates with the user. If the system meets uncertainty in any intermediate results or final prediction, the user is asked, using the LLM AI, for additional input to clarify or advise. The user provides clarification 460, such as text or other input to the LLM AI. The clarification is used to generate 430 another executable program 440 as an alteration.

The LLM AI is generative: able to call existing features (e.g., APIs 420) and/or generate a new hybrid function with a stack of existing function APIs 420 for a target task. The execution 440 generates results for the user request 410, which results are returned 460 to the user.

In one example, the user input 410 is a goal, stated to the LLM AI as "Quantify anatomy change between pre-operative image and acquired live image during intervention." The clinical workflow for such a goal includes 1) finding the anatomy in the pre-op image (e.g., CT image), 2) calculate all relavent measurements, 3) find the anatomy in live image (e.g., ultrasound image), 4) calculate all relavent measurements, and 5), for each measurement, compare and find the difference.

In a manual approach not using the LLM AI, the user 1) clicks ,load' to have preoperative (e.g., CT) image on screen, 2) clicks ,detect' panel, 3) clicks anatomy of interest (e.g., clicks a 'Mitral Valve' tab, 4) clicks structure of the anotomy (e.g., clicks ,Annulus' structure), 5) clicks ,visualize' to verify structure, 6) clicks 'measurements' to measure min/max diameter, area, perimeter, etc., 7) - 12) repeat same process for the live image (e.g., ultrasound image), and 13), for each measurement, compares whether they are different.

Using the LLM AI, the clinician types in text window: "how much has the mitral valve annulus changed since the previous aquired CT image?" The LLM AI automatically parses the user input and generates the executable code. The LLM AI auto-programs the medical imaging system to call the function APIs 'load', 'Detect', and 'Measure' already existing in the medical imaging system. Based on the generated program, the selected functions are called in the selected order. The second type of image may be extrapolated by the LLM AI as an ultraosund or other recent image. The goal of the AI auto-program is to call these APIs in the correct order and specify correct input parameters to achieve the target task.

Figure 6 shows a medical system, such as a medical imaging system. The medical system uses LLM AI 602 to receive sentence structure input and generate executable code 604 calling a selection of available functions 606 based on the input.

The medical system includes the display 620, memory 600, processor 610, and user input 630. The display 620, processor 610, user input 630, and/or memory 600 may be part of a computer, server, workstation, scanner, or another system for medical diagnosis, prognosis, and/or treatment of a patient based on medical images.

Additional, different, or fewer components may be provided. For example, a computer network is included for remote application of the LLM AI based on locally entered input and output results. As another example, a scanner, antenna, or front-end scanning hardware may be included for acquiring images, such as providing two scanners of different modalities.

The memory 600 is configured by formatting and/or the processor 610 to store information for a patient. For example, the memory 600 stores one or more medical images representing the patient for each of one or more imaging modalities. The memory 600 stores the LLM AI 602, such as the LLM AI 602 configured for medical imaging analysis or generating executable code for medical image processing. The LLM AI 602 as engineered, instructed, and/or prompted for the medical imaging context is stored in the memory 600. The LLM AI 602 as calibrated is stored. Alternatively, the LLM AI prior to engineering, instruction, prompting, and/or calibration is stored. Parts of the LLM AI 602 may be in different memories, such as where the memory 600 is a distributed memory.

The memory 600 is an external storage device, RAM, ROM, database, and/or a local memory (e.g., solid state drive or hard drive). The same or different non-transitory computer readable media may be used for the instructions and other data. The memory 600 may store instructions for operating the processor 610, generated program code 604, and/or available functions 606. The memory 600 may be implemented using a database management system (DBMS) and be a hard disk, RAM, or removable media. Alternatively, the memory 600 is internal to the processor 610 (e.g., cache). The memory 600 is formed from one device or a collection of devices, such as different memories storing different types of data.

The instructions for implementing the training (e.g., prompt engineering and/or calibration), application process (i.e., code generation for image processing for a patient), the methods, and/or the techniques discussed herein by the processor 610 are provided on non-transitory computer-readable storage media or memories, such as a cache, buffer, RAM, removable media, hard drive or other computer readable storage media (e.g., the memory 600). Computer readable storage media include various types of volatile and nonvolatile storage media. The functions, acts or tasks illustrated in the figures or described herein are executed in response to one or more sets of instructions stored in or on computer readable storage media. The functions, acts or tasks are independent of the particular type of instructions set, storage media, processor or processing strategy and may be performed by software, hardware, integrated circuits, firmware, micro code and the like, operating alone or in combination.

In one embodiment, the instructions are stored on a removable media device for reading by local or remote systems. In other embodiments, the instructions are stored in a remote location for transfer through a computer network. In yet other embodiments, the instructions are stored within a given computer, CPU, GPU, or system. Because some of the constituent system components and method steps depicted in the accompanying figures may be implemented in software, the actual connections between the system components (or the process steps) may differ depending upon the way the present embodiments are programmed.

The user input 630 is a keyboard, buttons, sliders, dials, trackball, mouse, touch pad, touch screen, microphone, and/or another device for user interaction with the medical system. The user input 630 is part of a graphics user interface for receiving user input to the LLM AI 602, such as text or audio in a sentence structure. The user input 630 is configured by the processor 610 and/or hardware to receive a sentence defining a user request with respect to one or more medical images of a patient. The request may be with respect to multiple images, such as where the images are part of a multi-modal image set (e.g., CT pre-operative image and ultrasound live image).

The processor 610 is a control processor, general processor, digital signal processor, graphics processing unit, application specific integrated circuit, field programmable gate array, artificial intelligence processor or accelerator, digital circuit, analog circuit, combinations thereof, or another now known or later developed device for implementing the LLM AI 602 and/or image processing. The processor 610 is a single device, a plurality of devices, or a network. For more than one device, parallel or sequential division of processing may be used. Different devices making up the processor 610 may perform different functions or the same function in parallel. The processor 610 operates pursuant to stored instructions, hardware, and/or firmware to perform various acts described herein.

In one embodiment, the processor 610 is configured to train the LLM AI 602. For example, the processor 610 receives the prompt from the user input 630, calibration feedback, context training data (e.g., database documents), instructions, ground truth data, and/or other LLM training data. The processor 610 implements the LLM AI to learn from the received input.

In another implementation, the processor 610 is configured to apply the LLM AI for a patient. The processor 610 receives the input sentence from the user and provides the input to the LLM AI 602. The processor 610 applies the LLM AI 602. The LLM AI 602 generates, and the processor 610 receives a sequence of calls (i.e., code 604) for APIs (i.e., selected functions 606) from the LLM AI 602 in response to the input of the sentence(s) to the LLM AI 602. The generated sequence uses any of various inputs, such as one or more images from one or more modalities (e.g., at least two images of a multi-modality set). The sequence was not previously available to the processor 610 and/or in the memory 600 but instead was generated by the LLM AI 602. The memory 600 was free of the sequence prior to generation. For a given patient and/or for a given procedure, the sequence does not exist until the LLM AI 602 generates the sequence.

The processor 610 is configured to implement the sequence using the medical image(s). The processor 610 executes the generated code 604. Based on this execution, the processor 610 calls the functions 606 included in the generated code 604 to image process. The execution occurs in an order and using information to provide a result or estimate for the user input.

The processor 610 may be configured to monitor confidence results from the called functions (e.g., APIs) during the implementation (execution) of the sequence. The processor 610 without using the LLM AI 602 or using the LLM AI 602 monitors the execution, such as monitoring results from any of the called APIs. For example, uncertainty or confidence in aggregation and/or for specific ones of the called APIs is monitored. The processor 610 provides or uses the LLM AI 602 to interact with a user when one of the confidence results is below a threshold. The LLM AI 602 may alter the generated code 604, such as by changing which APIs to call, changing an input or parameter of an API, changing an order of APIs, and/or making another change.

The processor 610 is configured to generate an image. An image showing the results of the called APIs (e.g., functions 606) of the generated code 604, the confidence information, and/or a final estimate (output answering the user request) is generated. Other information may be included in the image, such as one or more representations of the patient anatomy (e.g., a CT and/or ultrasound image).

The estimate (e.g., answer to the user input statement) is provided to assist in the medical procedure. For example, a measurement is generated to assist a physician in inserting a catheter the proper length, placing treatment (e.g., ablation or stent), or performing another intervention. As another example, landmark detection, segmentation, classification, and/or a measurement are generated to assist in diagnosis and/or prognosis.

The display 620 is a CRT, LCD, projector, plasma, printer, tablet, smart phone, or another now known or later developed display device for displaying the image. The display 620 is configured by loading an image to a display buffer or plane, which image is then displayed on a screen. Other configuration may be provided, such as configuring for display by printing. The display 620 is configured to display the answer to the user request derived from the implementation of the sequence (e.g., generated code 604).

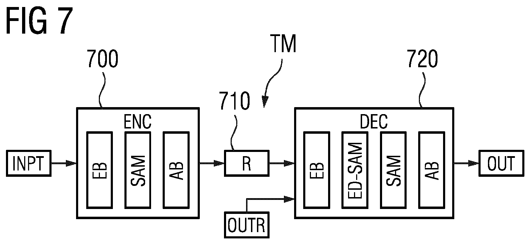

Figure 7 is shows an example transformer. The LLM AI is formed from one or more transformers. In Figure 7, a schematic representation of a transformer model TM is shown. The transformer model TM is configured to process a goal as input INPT and return a program as out-put OUT. The transformer architecture follows an encoder-decoder structure formed from an encoder ENC 700 and a decoder DEC 720. In brief, the task of the encoder ENC 700 is to map an input INPT to a sequence of continuous representations R 710, which is then fed into a decoder DEC 720. The decoder DEC 720 receives the representations R 710 together with the decoder output OUTR at a previous iteration to generate an out-put OUT.

The encoder ENC 700 of this embodiment is generally configured to transform individual data items of the input INPT into a numerical representation R 710. According to some examples, the numerical representation R 710 may take the form of a numerical vector for each data item of the input INPT. The representation R encodes how relevant a particular data item of the input INPT is with regard to other data items in the input INPT. To provide the numerical representation R 710, the encoder ENC 700 may include a plurality of blocks. A first block EB may be configured as an embedding block EB configured to bring the data items into a machine-readable form, the so-called embeddings, while preserving certain relations of the data items such as positional relations. In particular, the embeddings may be numerical vectors. The embeddings are fed into a second block SAB which may be designated as a self-attention block SAB. The self-attention block SAB implements a self-attention mechanism configured to determine how relevant a particular data item of the input INPT is with regard to other data items in the input INPT and modify the embeddings based on this information so as to generate attention vectors. According to some examples, the self-attention block SAB may be realized as neural network with one or more hidden layers. Said neural network may be trained to determine multiple attention vectors by data item and integrate these into one resulting attention vector. With regard to the latter, the self-attention block SAB may be configured to implement an additive or dot-product-based combination of individual attention vectors. The dimension of the attention vectors generated reflects how many data items are being compared in the self-attention block SAB. If, for instance, the self-attention block SAB is configured to compare 768 data items, the self-attention vector may have a dimension of 768 entries. The attention vectors are then fed into an adaptation block AB which is configured to map the attention vectors to a form which is suited for further processing (either by another self-attention block or the decoder or any other downstream processing).

The decoder DEC 720 of this embodiment may generally be configured to map the representation R 710 onto a desired output OUT. The decoder DEC 720 of this embodiment is configured to compute the output OUT based on the representations R 710 provided by the encoder ENC 700 and an output OUTR of a previous iteration. Like the encoder ENC 700, the decoder DEC 720 relies on a self-attention mechanism. Specifically, the decoder DEC 720 may include a plurality of blocks. Similar to the encoder ENC 700, a first block EB may be configured as an embedding block EB translating the previous output OUTR into embeddings. The embeddings are input to a self-attention block SAB configured to provide an attention vector of the previous output OUTR based on its embeddings. The attention vectors of the previous output OUTR are input into an encoder-decoder self-attention block ED-SAB together with the representations R 710 as provided by the encoder ENC 700. The encoder-decoder self-attention block ED-SAB is configured to map the representation to the attention vectors of the previous output OUTR so as to regressively improve the previous output OUTR. The output of the encoder-decoder self-attention block ED-SAB may be seen as attention vectors for data items in the input INPT and the vector space of the desired output OUT based on the previous output OUTR. These attention vectors are then fed into an adaptation block AB configured to map the attention vectors to a form suited for further processing (either by another self-attention block or by way of the generation of the final output).

The designation of the distinct blocks EB, SAB, ED_SAB, AB and the number of blocks shown in Figure 7 is to be construed by way of example and not as a limitation. Specifically, individual blocks EB, SAB, AB may be integrated to form one single block. In particular, all blocks may respectively be a neural network each with a plurality of layers. Moreover, there may be a plurality of stacks of self-attention blocks SAB and adaptation blocks AB in the encoder ENC 700, wherein the last adaptation block provides the final numerical representation R 710. Likewise, there may be a plurality of stacks of encoder-decoder self-attention blocks ED-SAB and adaptation blocks AB in the decoder DEC 720, wherein the last adaptation block AB provides the final output OUT. According to some examples, the decoder structure shown in Figure 7 may be replaced by a final adaptation block also referred to as "interpreter" configured to directly map the representation of the encoder on one or more learned outputs OUT which are human understandable.

In some instances, the encoders and/or decoders are composed of several corresponding encoding layers and decoding layers, respectively. Within each encoding and decoding layer preferably there is an attention mechanism. The attention mechanism, sometimes denoted as self-attention, relates data items (such as words or pixels) within a series of data items to other data items within said series. The self-attention mechanism for instance allows the model to examine a group of words within a sentence and determine the relative importance of other groups of words within that sentence for the group of words being examined. The encoder, in particular, may be configured to transform the input text into a numerical representation. The numerical representation may be a vector per input token [e.g., per word]. The encoder may be configured to implement an attention mechanism so that each vector of a token is affected by the other tokens in the input. In particular, the encoder may be configured such that the representations R 710 resolve the desired output of the transformer network TM.

The decoder, in particular, may be configured to transform an input into a sequence of output tokens. In particular, the decoder may be configured to implement a masked self-attention mechanism so that each vector of a token is affected only by the other tokens to one side of a sequence. Further, the decoder may be auto-regressive meaning in that intermediate results are fed back According to some examples, the input of the decoder is based on the output of the encoder or equivalent to the output of the encoder. Further, the transformer network may include a classification module configured to map the output of the encoder or decoder to a set of learned outputs.

Training of a transformer model according to some examples may happen in two stages, a pretraining and a fine-tuning stage. In the pretraining stage, a transformer model may be trained on a large corpus of data to learn the underlying semantics of the problem. Such pre-trained transformer models are available for different languages. For certain applications described herein, the fine-tuning may include further training the transformer network with medical texts with expert annotated meanings and/or medical ontologies such as RADLEX and/or SNOMED. With the latter, in particular, the transformer model according to some examples may learn typical relations and synonyms of medical expressions.

An advantage of transformer networks is that, due to the attention mechanism, transformer networks can efficiently deal with long-range dependencies in input data. Further, encoders used in transformer networks are capable of processing data in parallel, which saves computing resources in inference. Moreover, decoders of transformer networks, due the auto-regression, are able to iteratively generate a sequence of output tokens with great confidence.

Listed below are various Illustrative Embodiments. The Illustrative Embodiments summarize different combinations of aspects. Other combinations of any of the aspects with any other one or more of the aspects may be provided. Aspects from one type (e.g., method or system) may be used in another type (system or method).

Illustrative Embodiment 1: a method for decision making in a medical imaging system, the method comprising: acquiring a first medical image of a patient; receiving, by a large language model artificial intelligence (LLM AI), user input identifying a goal with respect to the first medical image; generating, by the LLM AI, an executable program calling multiple analysis functions of the medical imaging system to achieve the goal; executing, by an image processor of the medical imaging system, the executable program, at least a first one of the multiple analysis functions called by the executing of the executable program operating on the first medical image; and displaying an estimate of the goal, the estimate being derived from results of the executing.

Illustrative Embodiment 2: The method of Illustrative Embodiment 1 wherein acquiring comprises acquiring the first medical image and a second medical image, the first and second medical images being from first and second, different, and medical imaging modalities, wherein at least a second one of the multiple analysis functions called by executing the operating on the second medical image.

Illustrative Embodiment 3: The method of any of Illustrative Embodiments 1-2 wherein receiving comprises receiving the user input as a selection from a user interface, text, or audio.

Illustrative Embodiment 4: The method of any of Illustrative Embodiments 1-3 wherein receiving comprises receiving a question in a sentence structure.

Illustrative Embodiment 5: The method of any of Illustrative Embodiments 1-4 wherein generating the executable program comprises generating computer code.

Illustrative Embodiment 6: The method of any of Illustrative Embodiments 1-5 wherein generating comprises generating the executable program comprises generating the executable program calling the multiple analysis functions as application programming interfaces of the medical imaging system.

Illustrative Embodiment 7: The method of Illustrative Embodiment 6 wherein the application programming interfaces comprise image processing for loading the first image, detection of a landmark, and measurement with respect to the landmark.

Illustrative Embodiment 8: The method of any of Illustrative Embodiments 1-7 wherein generating the executable program comprises generating the executable program with a selection of the multiple analysis functions as a sub-set from a group of available analysis functions and an order of the multiple analysis functions based on input parameters of the multiple analysis functions.

Illustrative Embodiment 9: The method of any of Illustrative Embodiments 1-8 wherein generating comprises generating by the LLM AI where the LLM AI was prompt-engineered with a database of workflow examples of uses of the medical imaging system and available analysis functions of the medical imaging system.

Illustrative Embodiment 10: The method of Illustrative Embodiment 9 wherein generating comprises generating by the LLM AI where the LLM AI was prompted engineered with ground truth examples from a prompt describing the database, an instruction to generate the executable program, and a limitation.

Illustrative Embodiment 11: The method of any of Illustrative Embodiments 1-10 wherein generating comprises generating by the LLM AI where the LLM AI was calibrated from (1) questions for workflow examples and (2) positive and/or negative feedback for example executable programs generated by the LLM AI for the questions.

Illustrative Embodiment 12: The method of any of Illustrative Embodiments 1-11 further comprising: monitoring, by the LLM AI, confidence information from the analysis functions during the executing; and altering, by the LLM AI, the executable program based on the confidence information being below a threshold.

Illustrative Embodiment 13: The method of Illustrative Embodiment 12 wherein altering comprises the LLM AI interacting with the user and altering in response to clarification and/or instruction from the user.

Illustrative Embodiment 14: The method of any of Illustrative Embodiments 1-13 further comprising determining a sensitivity of the estimate, wherein displaying comprises displaying the estimate and the sensitivity.

Illustrative Embodiment 15: The method of any of Illustrative Embodiments 1-14 wherein generating comprises generating the executable program as a program not pre-existing in the medical imaging system.

Illustrative Embodiment 16: A medical system comprising: a memory configured to store a large language model artificial intelligence (LLM AI) calibrated for medical imaging; a user input configured to receive a sentence defining a user request with respect to a medical image of a patient; a processor configured to input the sentence to the LLM AI, to receive a sequence of calls for application programming interfaces from the LLM AI generated in response to the input, and to implement the sequence using the medical image; and a display configured to display an answer to the user request derived from the implementation of the sequence.

Illustrative Embodiment 17: The medical system of Illustrative Embodiment 16 wherein the medical image is part of a multi-modal image set, and wherein the generated sequence of calls uses the medical image and another image of the multi-modal image set.

Illustrative Embodiment 18: The medical system of any of Illustrative Embodiments 16-17 wherein the LLM AI generates the sequence where the memory is free of the sequence prior to the generation by the LLM AI.

Illustrative Embodiment 19: The medical system of any of Illustrative Embodiments 16-18 wherein the processor is configured to monitor confidence results from the application programming interfaces during the implementation of the sequence and to provide for the LLM AI to interact with a user when one of the confidence results is below a threshold.

Illustrative Embodiment 20: A method for decision making in a medical imaging system, the method comprising: programming the medical imaging system by a large language model to operate on medical images of different modalities using available functions of the medical imaging system to answer a user request; and displaying an answer to the user request.

Various improvements described herein may be used together or separately. Although illustrative embodiments of the present invention have been described herein with reference to the accompanying drawings, it is to be understood that the invention is not limited to those precise embodiments, and that various other changes and modifications may be affected therein by one skilled in the art without departing from the scope of the invention.

## Claims

1. A method for decision making in a medical imaging system, the method comprising:
acquiring (200) a first medical image of a patient;
receiving (210), by a large language model artificial intelligence, LLM AI, (602), user input identifying a goal with respect to the first medical image;
generating (220), by the LLM AI (602), an executable program (604) calling multiple analysis functions (606) of the medical imaging system to achieve the goal;
executing (230), by an image processor (610) of the medical imaging system, the executable program (604), at least a first one of the multiple analysis functions (606) called by the executing (230) of the executable program (604) operating on the first medical image; and
displaying (270) an estimate of the goal, the estimate being derived from results of the executing (230).

2. The method of claim 1, wherein acquiring (200) comprises acquiring (200) the first medical image and a second medical image, the first and second medical images being from first and second, different, and medical imaging modalities, wherein at least a second one of the multiple analysis functions (606) called by executing (230) the operating on the second medical image.

3. The method of claim 1 or 2, wherein receiving (210) comprises receiving (210) the user input as a selection from a user interface, text, or audio and/or wherein receiving (210) comprises receiving (210) a question in a sentence structure.

4. The method of any one of claims 1 - 3, wherein generating (220) comprises generating (220) the executable program (604) comprises generating (220) the executable program (604) calling the multiple analysis functions (606) as application programming interfaces (420) of the medical imaging system.

5. The method of claim 4, wherein the application programming interfaces (420) comprise image processing for loading the first image, detection of a landmark, and measurement with respect to the landmark.

6. The method of any one of claims 1 - 5, wherein generating (220) the executable program (604) comprises at least one of:
generating (220) computer code;
generating (220) the executable program (604) with a selection of the multiple analysis functions (606) as a sub-set from a group of available analysis functions (606) and an order of the multiple analysis functions (606) based on input parameters of the multiple analysis functions (606);
generating (220) the executable program (604) as a program (604) not pre-existing in the medical imaging system;
generating (220) the executable program (604) by the LLM AI (602), where the LLM AI (602) was prompt-engineered with a database of workflow examples of uses of the medical imaging system and available analysis functions (606) of the medical imaging system;
generating (220) the executable program (604) by the LLM AI (602), where the LLM AI (602) was prompted engineered with ground truth examples from a prompt describing the database, an instruction to generate the executable program (604), and a limitation; and
generating (220) the executable program (604) by the LLM AI (602), where the LLM AI (602) was calibrated from (1) questions for workflow examples and (2) positive and/or negative feedback for example executable program (604)s generated by the LLM AI (602) for the questions.

7. The method of any one of claims 1 - 6, further comprising:
monitoring (240), by the LLM AI (602), confidence information from the analysis functions (606) during the executing (230); and/or
altering (250), by the LLM AI (602), the executable program (604) based on the confidence information being below a threshold.

8. The method of claim 7, wherein altering (250) comprises the LLM AI (602) interacting with the user and altering (250) in response to clarification and/or instruction from the user.

9. The method of any one of claims 1 - 8, further comprising determining a sensitivity of the estimate, wherein displaying (270) comprises displaying (270) the estimate and the sensitivity.

10. A medical system comprising:
a memory (600) configured to store a large language model artificial intelligence, LLM AI, (602) calibrated for medical imaging;
a user input (630) configured to receive a sentence defining a user request with respect to a medical image of a patient;
a processor (610) configured to input the sentence to the LLM AI (602), to receive a sequence of calls for application programming interfaces (420) from the LLM AI (602) generated in response to the input, and to implement the sequence using the medical image; and
a display (620) configured to display an answer to the user request derived from the implementation of the sequence.

11. The medical system of claim 10, wherein the processor (610) is further configured to execute the steps according to the method of any one of claims 1 - 9, the first medical image being the medical image of the patient, the goal being comprised in the sentence, the multiple analysis functions (606) being called by the executable program (604) as the application programming interfaces (420), and the estimate of the goal being comprised in the answer to the user request.

12. The medical system of claim 10 or 11, wherein the medical image is part of a multi-modal image set, and wherein the generated sequence of calls uses the medical image and another image of the multi-modal image set.

13. The medical system of any one of claims 10 - 12 wherein the LLM AI (602) generates the sequence where the memory (600) is free of the sequence prior to the generation by the LLM AI (602).

14. The medical system of any one claims 10 - 13, wherein the processor (610) is configured to monitor confidence results from the application programming interfaces (420) during the implementation of the sequence and to provide for the LLM AI (602) to interact with a user when one of the confidence results is below a threshold.

15. A method for decision making in a medical imaging system, the method comprising:
programming (430) the medical imaging system by a large language model (602) to operate on medical images of different modalities using available functions (606) of the medical imaging system to answer a user request; and
displaying (270) an answer to the user request,
wherein programming, in particular, includes loading the executable program generated through the method of any one of claims 1 - 9 into a memory of the medical imaging system, the goal being comprised in the user request.
